# EUROPEAN PATENT SPECIFICATION

(11) **EP 3 251 725 B1**
(45) Date of publication and mention of the grant of the patent: **12.12.2018**
(21) Application number: 16172047.9
(22) Date of filing: 30.05.2016
(51) Int. Cl.: A61N 5/06, A61N 5/00

(54) **DEVICE FOR RADIATING UV AND/OR IR IN A HUMID ROOM**
VORRICHTUNG ZUR ABSTRAHLUNG VON UV UND/ODER IR IN EINEM FEUCHTEN RAUM
DISPOSITIF D'IRRADIATION DE RAYONS ULTRAVIOLETS ET/OU IR DANS UNE SALLE HUMIDE

(43) Date of publication of application: 06.12.2017
(73) Proprietor: Sunshower B.V., 1101 BB Amsterdam (NL); Wegdam, Pieter Merijn, 1402 ER Bussum (NL)
(72) Inventor: Wegdam, Pieter Merijn, 1402 ER Bussum (NL); Seen, Elmer, 1101 BB Amsterdam (NL); Jong, Jesse, 1101 BB Amsterdam (NL)
(74) Representative: V.O.

(56) References cited:
- EP-A1- 0 208 392
- WO-A1-2008/140315
- DE-A1- 3 433 603
- DE-A1- 3 708 872
- US-A1- 2016 136 385

## Description

The invention refers to a device arranged for radiating UV and/or IR radiation to a human body, in particular in a humid room like a bath of shower room.

Such device is known as such from earlier patent applications WO2008140315A1 and WO2004004830A1 of the same applicant.

The known device comprises an upright housing comprising one or more radiation units each including one or more radiation sources and one or more reflectors, said reflectors being covered by a first radiation transmitting wall and a second radiation transmitting wall, parallel to the first radiation transmitting wall and spaced apart from it. The device includes a cooling circuit for cooling the radiation units and/or the first and/or second radiation transmitting wall, comprising
- a cooling channel inlet at the bottom part of the device,
- a front cooling channel, extending from said channel inlet and including the space between said first and second radiation transmitting walls, and
- a rear cooling channel, connected to the front cooling channel through an interconnection part at the top side of the device, including a space between the radiation units and the rear and/or side walls of the upright housing,
the rear cooling channel being connected to an exhaust ventilator at the bottom side of the device, arranged to remove, via a channel outlet, cooling air sucked by the ventilator into the cooling circuit via said channel inlet and flowing through the front and rear channels meanwhile cooling the radiation units.

One aim of the present invention is to improve the device such that the entry of humid air from the device's environment, viz. the humid room, into the inside of the device via the inlet and/or the outlet of the cooling channel, is prevented when the device is switched off, thus preventing the formation of condensation inside the device and thus malfunctioning of the device on long term.

Another aim is to reduce the temperature of the front radiation transmitting wall, to prevent that the user can burn himself or herself. The air flow in the device is improved, which results in heat reduction of both radiation transmitting walls (also called filter plates), which results in lower temperatures at the front side of the device.

Yet another aim is to improve the device's electrical security and reliability (double insulated, class II and protected against water jets, IPX5).

According to the present invention a double-acting protective valve is provided which is arranged to close (and to keep closed) both the channel inlet and the channel outlet when the exhaust ventilator is off, and/or to open the channel inlet and the channel outlet (only) when the exhaust ventilator is on. As a result of this entering or penetration of splash water and humid air (moisture) into the inside of the device through the inlet and outlet of the cooling channel when the ventilator is off, is prevented, viz. by shutting both the inlet and the outlet. When the ventilator is on, however, entering or penetration of splash water and humid air (moisture) into the inside of the device through the inlet will not cause problems in the inside of the device as, as long the radiation sources in the device are switched on, the inside of the device has such a high temperature that the splash water and moisture will evaporate and exhausted via the cooling channel's outlet, driven by the exhaust ventilator.

Preferably, the protective valve comprises an inlet member and an outlet member, arranged to open and close the channel inlet and channel outlet respectively under control of the air flow originated by the ventilator. To that end the inlet member and the outlet member may be interconnected and enabled to seesaw around pivoting means connected to the device's housing. To close the inlet and outlet members as soon as the air flow originated by the ventilator stops, the center of gravity of the interconnected inlet member and outlet member is at the side of the channel inlet and wherein, to open the inlet and outlet members as soon as the ventilator starts, the outlet member comprises an air flow receiving deflection member, arranged to actuate, by its deflection, the outlet member and the interconnected inlet member by the air flow received from the ventilator and/or the channel outlet.

A stop member may be provided for restricting the deflection of the deflection member and/or outlet member, in particular in view of shielding the inlet and outlet openings against direct and indirect splash and jet water.

Hereinafter the invention will be elucidated on the basis of some exemplary embodiments, with reference to some figures.
- Figure 1: shows an exemplary embodiment of a device according to the invention;
- Figure 2: shows a detail of the same exemplary embodiment in two operational situations;
- Figure 3: shows the same exemplary embodiment during test for IPX5 qualification.

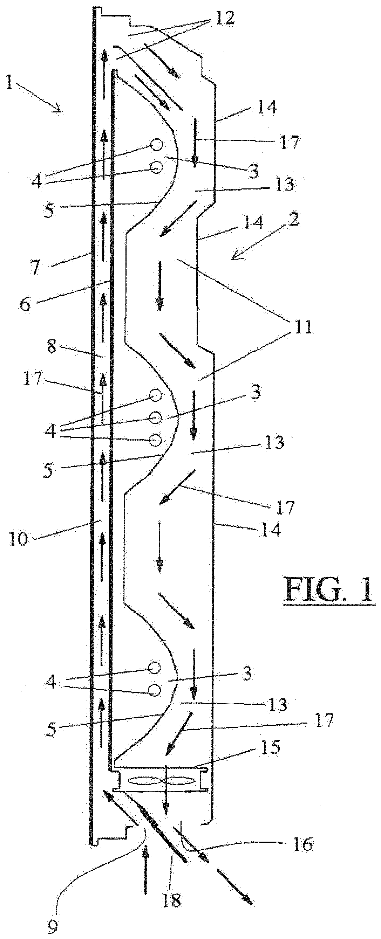

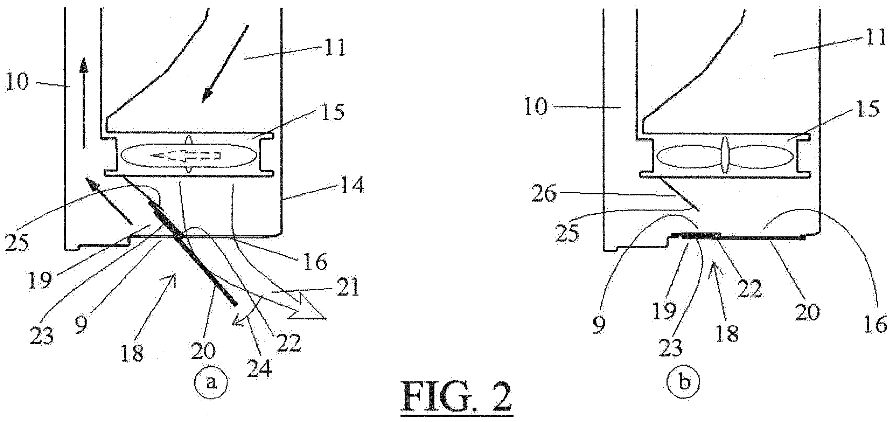

Figures 1 and 2 illustrate schematically a device 1 arranged for radiating UV and/or IR radiation to a human body, which device may be located in the corner of a bath of shower room. The device comprises an upright housing 2 comprising radiation units 3 each including radiation sources 4 and reflectors 5, said reflectors 5 being covered by a first radiation transmitting wall (or filter plate) 6 and a second radiation transmitting wall (or filter plate) 7, parallel to the first radiation transmitting wall 6 and spaced apart (8) from it.

The device includes a cooling circuit for cooling the radiation units 3 and/or the first radiation transmitting wall 6 and/or the second radiation transmitting wall 7, comprising
- a cooling channel inlet 9 at the bottom part of the device 1,
- a front cooling channel 10, extending from said channel inlet 9 and including the space 8 between said first and second radiation transmitting walls 6 and 7, and
- a rear cooling channel 11, connected to the front cooling channel 10 through an interconnection channel part 12 at the top side of the device 1, which rear cooling channel 11 includes the space 13 between the radiation units 3 (especially their reflectors 5) and the rear and/or side walls 14 of the upright housing 2.

The rear cooling channel 11 is connected to (debouches into) an exhaust ventilator 15 at the bottom side of the device 1, arranged to remove, via a channel outlet 16, cooling air 17 sucked by the ventilator 15 into the cooling circuit 10,12,11 via said channel inlet 9 and flowing through the front and rear channels 10,11 meanwhile cooling the radiation units 3.

A double-acting protective valve 18 is provided which is arranged to close both the channel inlet 9 and the channel outlet 16 when the (electrical) exhaust ventilator 15 is (switched) off, and/or to open the channel inlet 9 and the channel outlet 16 when the exhaust ventilator 15 is (switched) on (under control of control means, not shown in the figures).

As figure 2 illustrates more in detail, the protective valve 18 comprises an inlet member 19 and an outlet member 20, arranged to open and close the channel inlet 9 and channel outlet 10 respectively under control of the air flow 21 originated by the ventilator 15. The inlet member 19 and the outlet member 20 are interconnected and enabled to seesaw around pivoting means, e.g. axle shafts 22 journalled in a bearing provided in or to the device's housing 14.

In order to close the inlet and outlet members 9 and 16 as soon as the air flow 21 originated by the ventilator 15 stops, the center of gravity of the interconnected inlet member 9 and outlet member 16 is at the side of the channel inlet 9, by means of a load element 23, giving the seesawing valve 18 a preferred (default) closed position. In order to open the inlet and outlet members 19 and 20 of the valve 18 as soon as the ventilator 15 starts, the outlet member 20 comprises or incorporates an air flow receiving deflection member (also labelled with 20), arranged to actuate, by its rotational deflection (see arrow 24), the outlet member 20 and the interconnected inlet member 19, driven by the air flow 21 received from the ventilator 15 and/or the channel outlet 16.

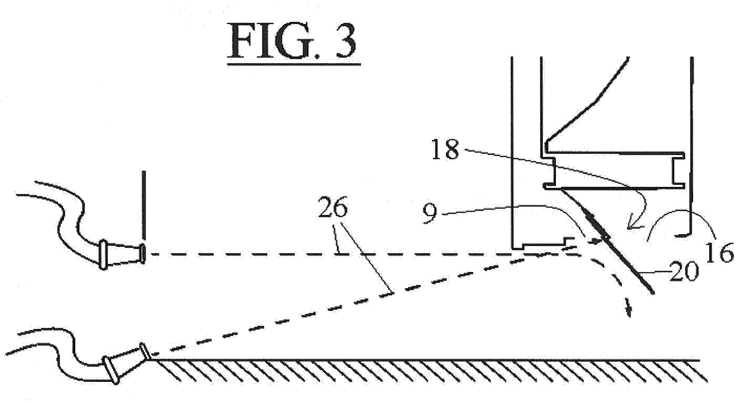

The device 1 comprises a stop member 25 which is arranged for restricting the deflection 24 of the deflection member/outlet member 20, in particular in view of shielding the inlet and outlet openings against direct and indirect splash and jet water, which has been illustrated in figure 3, showing the device 1 under test for IPX5 qualification and illustrating that water splashes and jets 26 will be deflected by the (open) the deflection member/outlet member 20, thus prevent-ing the water to enter into the device through the cooling channel's inlet 9 and/or outlet 16.

It is noted that the figures are only schematic representations of embodiments of the invention that are given by way of non-limiting example. In the figures, the same or corresponding parts are designated with the same reference numerals.

For the purpose of clarity and a concise description, features are described herein as part of the same or separate embodiments, however, it will be appreciated that the scope of the invention may include embodiments having combinations of all or some of the features described. It may be understood that the embodiments shown have the same or similar components, apart from where they are described as being different.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to an advantage.

Many variants will be apparent to the person skilled in the art. All variants are understood to be comprised within the scope of the invention defined in the following claims.

## Claims

1. Device (1) arranged for radiating UV and/or IR radiation to a human body, the device comprising an upright housing (2) comprising one or more radiation units (3) each including one or more radiation sources (4) and one or more reflectors (5), said reflectors being covered by a first radiation transmitting wall (6) and a second radiation transmitting wall (7), parallel to the first radiation transmitting wall and spaced apart from it;
the device including a cooling circuit for cooling the radiation units and/or the first and/or the second radiation transmitting walls (6,7), comprising
- a cooling channel inlet (9) at the bottom part of the device,
- a front cooling channel (10), extending from said channel inlet and including the space (8) between said first and second radiation transmitting walls, and
- a rear cooling channel (11), connected to the front cooling channel through an interconnection part (12) at the top side of the device, including a space (13) between the radiation units and the rear and/or side walls (14) of the upright housing,
- an exhaust ventilator (15) at the bottom side of the device,
the rear cooling channel being connected to said exhaust ventilator (15) and arranged to remove, via a channel outlet (16), cooling air (17) sucked by the ventilator into the cooling circuit via said channel inlet and flowing through the front and rear channels meanwhile cooling the radiation units,
**characterized by**
a double-acting protective valve (18) arranged to close both the channel inlet and the channel outlet when the exhaust ventilator is off, and/or to open the channel inlet and the channel outlet when the exhaust ventilator is on.

2. Device according to claim 1, said protective valve comprising an inlet member (19) and an outlet member (20), arranged to open and close the channel inlet and channel outlet respectively under control of the air flow (21) originated by the ventilator.

3. Device according to claim 2, wherein the inlet member and the outlet member are interconnected and enabled to seesaw around pivoting means (22).

4. Device according to claim 3, wherein the center of gravity of the interconnected inlet member and outlet member is at the side of the channel inlet and wherein the outlet member comprises an air flow receiving deflection member (20), arranged to actuate, by its deflection (24), the outlet member and the interconnected inlet member by the air flow received from the ventilator and/or the channel outlet.

5. Device according to claim 3 or 4, comprising a stop member (25) arranged for restricting the deflection of the deflection member and/or outlet member.

## Patentansprüche

1. Vorrichtung (1), angeordnet zur Abstrahlung von UV- und/oder IR-Strahlen auf einen menschlichen Körper, die Vorrichtung umfassend ein aufrechtes Gehäuse (2) umfassend eine oder mehrere Strahlungseinheiten (3), jede mit einer oder mehreren Strahlungsquellen (4) und einen oder mehrere Reflektoren (5), die Reflektoren bedeckt von einer ersten strahlungsübertragenden Wand (6) und einer zweiten strahlungsübertragenden Wand (7), parallel zu der ersten strahlungsübertragenden Wand und beabstandet davon, die Vorrichtung umfassend einen Kühlkreis zum Kühlen der Strahlungseinheiten und/oder der ersten und/oder der zweiten strahlungsübertragenden Wände (6, 7), umfassend
- einen Kühlkanaleinlass (9) am unteren Teil der Vorrichtung,
- einen vorderen Kühlkanal (10), verlaufend von dem Kanaleinlass und umfassend den Raum (8) zwischen den ersten und zweiten Strahlungsübertragenden Wänden, und
- einen hinteren Kühlkanal (11), verbunden mit dem vorderen Kühlkanal durch ein Verbindungsteil (12) an der Oberseite der Vorrichtung, umfassend einen Raum (13) zwischen den Strahlungseinheiten und den hinteren und/oder seitlichen Wänden (14) des aufrechten Gehäuses,
- einen Abzugslüfter (15) an der Unterseite der Vorrichtung, der hintere Kühlkanal verbunden mit dem Abzugslüfter (15) und angeordnet, um über einen Kanalauslass (16) Kühlluft (17), angesaugt von dem Lüfter in den Kühlkreis über den Kanaleinlass und strömend durch die vorderen und hinteren Kanäle während des Kühlens der Strahlungseinheiten zu entfernen,
**gekennzeichnet durch** ein doppelt wirkendes Schutzventil (18), angeordnet zum Schließen von sowohl dem Kanaleinlass als auch dem Kanalauslass, wenn der Abzugslüfter abgeschaltet ist, und/oder zu Öffnen des Kanaleinlasses und des Kanalauslasses, wenn der Abzugslüfter eingeschaltet ist.

2. Vorrichtung nach Anspruch 1, das Schutzventil umfassend ein Einlasselement (19) und ein Auslasselement (20), angeordnet zum Öffnen und Schließen des Kanaleinlasses bzw. des Kanalauslasses unter Steuerung des Luftstroms (21), hervorgebracht von dem Lüfter.

3. Vorrichtung nach Anspruch 2, wobei das Einlasselement und das Auslasselement miteinander verbunden und aktiviert sind, sich um Drehmittel (22) herum hin und her zu bewegen.

4. Vorrichtung nach Anspruch 3, wobei das Schwerkraftzentrum des miteinander verbundenen Einlasselements und Auslasselements sich an der Seite des Kanaleinlasses befindet und wobei das Auslasselement ein luftstromempfangendes Ablenkelement (20) umfasst, angeordnet, um durch seine Ablenkung (24) das Auslasselement und das damit verbundene Einlasselement durch den Luftstrom, empfangen von dem Lüfter und/oder dem Kanalauslass, zu aktivieren.

5. Vorrichtung nach Anspruch 3 oder 4, umfassend ein Anschlagelement (25), angeordnet zum Beschränken der Ablenkung des Ablenkelements und/oder des Auslasselements.

## Revendications

1. Dispositif (1) agencé pour émettre un rayonnement UV et/ou IR vers un corps humain, le dispositif comprenant un boîtier vertical (2) comprenant une ou plusieurs unités de rayonnement (3) chacune incluant une ou plusieurs sources de rayonnement (4) et un ou plusieurs réflecteurs (5), lesdits réflecteurs étant recouverts par une première paroi de transmission de rayonnement (6) et une seconde paroi de transmission de rayonnement (7) parallèle à la première paroi de transmission de rayonnement et espacée de celle-ci; le dispositif comprenant un circuit de refroidissement pour refroidir les unités de rayonnement et/ou la première et/ou seconde parois de transmission de rayonnement (6,7), comprenant
- une entrée de canal de refroidissement (9) dans la partie inférieure du dispositif,
- un canal de refroidissement avant (10), s'étendant depuis ladite entrée de canal et comprenant l'espace (8) entre lesdites première et seconde parois de transmission de rayonnement, et
- un canal de refroidissement arrière (11) relié au canal de refroidissement avant par une partie d'interconnexion (12) sur le sommet du dispositif, comprenant un espace (13) entre les unités de rayonnement et les parois arrière et/ou latérales (14) du boîtier vertical,
- un ventilateur d'évacuation (15) sur la face formant le fond du dispositif, le canal de refroidissement arrière étant relié audit ventilateur d'évacuation (15) et étant agencé pour enlever, via une sortie de canal (16), l'air de refroidissement (17) aspiré par le ventilateur dans le circuit de refroidissement via ladite entrée de canal et traversant les canaux avant et arrière en refroidissant les unités de rayonnement, **caractérisé en ce que** une valve de protection à double effet (18) agencée pour fermer à la fois l'entrée du canal et la sortie du canal lorsque le ventilateur d'extraction est éteint, et/ou ouvrir l'entrée du canal et la sortie du canal lorsque le ventilateur d'évacuation est activé.

2. Dispositif selon la revendication 1, ladite valve de protection comprenant un organe d'entrée (19) et un organe de sortie (20), est agencée pour ouvrir et fermer respectivement l'entrée et la sortie du canal sous contrôle du flux d'air (21) issu du ventilateur.

3. Dispositif selon la revendication 2, dans lequel l'élément d'entrée et l'élément de sortie sont interconnectés et peuvent basculer autour des moyens de pivotement (22).

4. Dispositif selon la revendication 3, dans lequel le centre de gravité de l'élément d'entrée et de sortie interconnectés est du côté de l'entrée de canal et dans lequel l'élément de sortie comprend un élément de déflexion de l'écoulement d'air reçu (20), agencé pour actionner, par la déflection (24) du flux d'air reçu du ventilateur et/ou de la sortie du canal l'élément de sortie et l'élément d'entrée interconnectés.

5. Dispositif selon la revendication 3 ou 4, comprenant un organe d'arrêt (25) agencé pour limiter la déflexion de l'élément de déflexion et/ou de l'élément de sortie.
